# EUROPEAN PATENT APPLICATION

(11) **EP 4 837 545 A1**
(43) Date of publication of application: **11.11.2026**
(21) Application number: 25221468.9
(22) Date of filing: 08.12.2025
(51) Int. Cl.: A61F 9/06, B23K 9/32

(54) **WELDING MASK**

(30) Priority: 07.05.2025 KR 20250059426
(71) Applicant: Otos Wing Co., Ltd., Seoul 08521 (KR)
(72) Inventor: HUH, Moon Young, 08521 Seoul (KR); HUH, Sung Won, 06084 Seoul (KR)
(74) Representative: Ström & Gulliksson AB

(57) **Abstract**

A welding mask having an image capturing function for welding work includes a main body that is wearable on the face of a user, a viewing window disposed on the main body and corresponding to the left and right eyes of the user, and a camera module mounted on the main body and configured to capture an external image located in a view of the user, wherein the camera module includes a lens barrel in which a lens unit is built, and a focus adjustment unit having one side connected to the lens barrel and the other side connected to the one side and exposed to an inside of the main body, the focus adjustment unit being configured to move the lens unit along an optical center axis as the lens barrel is rotated by manipulating the other side.

## Description

### BACKGROUND

### 1. Field

One or more embodiments relate to a welding mask.

### 2. Description of the Related Art

Protective equipment such as welding masks is generally used to protect users from light and high heat generated during welding work.

According to the related art, when wearing a welding mask, the user could only check the welding progress through the welding mask, and thus, in order to check various information for welding, such as the conditions set on a welding device, the user had to remove the welding mask and check with the naked eye, which was inconvenient.

In addition, welding masks according to the related art lacked the means to externally transmit or record specific image information about weld beads and molten pool at the welding work site, which limited their use in quality control, training materials, and remote monitoring.

In addition, since an imaging device was installed separately from the welding mask in the related art, there were problems such as mismatch between a visual field and an image, increased equipment weight, and increased cable complexity.

### SUMMARY

One or more embodiments provide a welding mask that may provide a stable and accurate view to a user during welding work to improve welding accuracy, and that may enhance usability by capturing, recording, and transmitting images of the welding work in real time.

One or more embodiments also provide a welding mask that may prevent focus shake caused by vibration or external impact occurring during welding work by allowing a user to adjust a camera focus to an actual view and lock and maintain the adjusted focus, and that thus may improve workability by maintaining a constant focus state during similar work.

However, the objectives are examples, and the scope of the present disclosure is not limited thereto.

According to one or more embodiments, provided is a welding mask including a main body that is wearable on the face of a user, a viewing window disposed on the main body and corresponding to the left and right eyes of the user, and a camera module mounted on the main body and configured to capture an external image located in a view of the user, wherein the camera module includes a lens barrel in which a lens unit is built, and a focus adjustment unit having one side connected to the lens barrel and the other side connected to the one side and exposed to an inside of the main body, the focus adjustment unit being configured to move the lens unit along an optical center axis as the lens barrel is rotated by manipulating the other side.

In an embodiment of the present disclosure, the focus adjustment unit may include a first gear coupled to an outer circumferential surface of the lens barrel, a second gear engaged with the first gear, and an operating element connected to the second gear via a shaft, exposed to the inside of the main body, and being rotatable by the user.

In an embodiment of the present disclosure, the first gear and the second gear may each include a spur gear, and the first gear and the second gear may rotate in opposite directions to each other.

In an embodiment of the present disclosure, the camera module may further include a locking unit arranged on one side of the operating element to restrict rotation of the operating element.

In an embodiment of the present disclosure, a plurality of gear teeth may be continuously formed on an outer circumferential surface of the operating element, and the locking unit may have a locking projection that is insertable between a plurality of gear teeth and the locking unit may be moved linearly back and forth in a radial direction of the operating element.

In an embodiment of the present disclosure, the camera module may be positioned above the viewing window, and an optical center axis of the camera module and a viewing center axis of the viewing window may have a preset angle therebetween.

In an embodiment of the present disclosure, an optical center of the camera module and a viewing center of the left and right eyes of the user may have a preset distance therebetween.

In an embodiment of the present disclosure, the welding mask may further include a battery detachably coupled to one side of the camera module and configured to supply power to the camera module.

In an embodiment of the present disclosure, the viewing window may include a magnifying glass unit corresponding to the left and right eyes of the user, a light irradiation unit positioned in front of the magnifying glass unit, and a cover unit arranged in front of the light irradiation unit.

In addition to the details described above, other aspects, features, and advantages will be clarified from the following drawings, claims, and detailed description.

### BRIEF DESCRIPTION OF THE DRAWINGS

The above and other aspects, features, and advantages of certain embodiments of the disclosure will be more apparent from the following description taken in conjunction with the accompanying drawings in which:
FIG. 1 is a perspective view illustrating a welding mask according to an embodiment of the present disclosure;
FIG. 2 is a rear view of FIG. 1;
FIG. 3 is a schematic side view showing a state in which a user wears the welding mask of FIG. 1;
FIG. 4 is a partial exploded perspective view of FIG. 1;
FIG. 5 is a perspective view illustrating a camera module of FIG. 4;
FIG. 6 is an exploded perspective view of FIG. 5;
FIG. 7 is a rear view of FIG. 5;
FIG. 8 is a cross-sectional view taken along line I-I' of FIG. 5;
FIG. 9 is a diagram partially illustrating a camera module of FIG. 5;
FIG. 10A is a diagram illustrating region 'A' of FIG. 7;
FIG. 10B is a diagram illustrating a state in which a rotational operation of an operating element is restricted by a locking unit of FIG. 10A;
FIG. 11 is a diagram for describing the relationship between a vertical angle of view of a camera module and a view of a user, according to an embodiment of the present disclosure; and
FIG. 12 is a diagram illustrating a horizontal angle of view of a camera module, according to an embodiment of the present disclosure.

### DETAILED DESCRIPTION

Embodiments will be described below in more detail with reference to the accompanying drawings. Those components that are the same or are in correspondence are rendered the same reference numeral regardless of the figure number, and redundant explanations are omitted.

As the present disclosure allows for various changes and numerous embodiments, particular embodiments will be illustrated in the drawings and described in detail in the written description. The effects and features of the embodiments, and ways to achieve them will become apparent by referring to the details that are described later in detail with reference to the drawings. However, the embodiments may have different forms and should not be construed as being limited to the descriptions set forth herein.

Elements not related to description are omitted in the drawings for clear description of the present disclosure, and like reference numerals in the drawings denote like elements throughout the specification.

While such terms as "first," "second," etc., may be used to describe various components, such components are not limited to the above terms. The terms are used only to distinguish one component from another.

An expression used in the singular encompasses the expression of the plural, unless it has a clearly different meaning in the context.

In the present specification, it is to be understood that the terms such as "including," "having," and "comprising" are intended to indicate the existence of the features or components disclosed in the specification, and are not intended to preclude the possibility that one or more other features or components may exist or may be added.

It will be understood that when a unit, region, or component is referred to as being "formed on" another layer, region, or component, it may be directly or indirectly formed on the other layer, region, or component. That is, for example, intervening units, regions, or components may be present.

It will be understood that when an element is referred to as being "connected" or "coupled" to another element, it may be directly connected or coupled to the other element or intervening elements may be present.

Sizes of components in the drawings may be exaggerated for convenience of description. In other words, since sizes and thicknesses of components in the drawings are arbitrarily illustrated for convenience of description, the following embodiments are not limited thereto.

The terms such as "on", "above", "under", "below" used in this specification are only used to easily describe the relationship between each component shown in the drawings, and do not limit the direction in which each component is arranged.

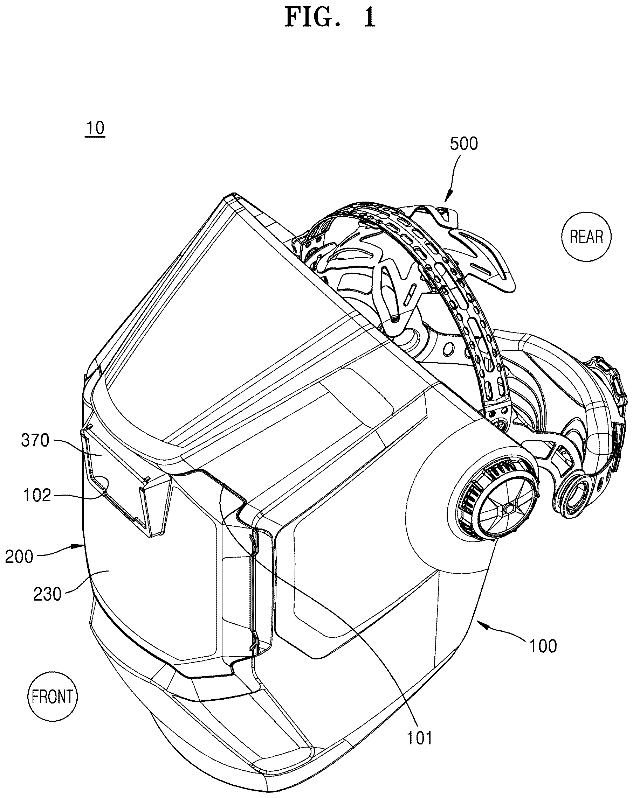

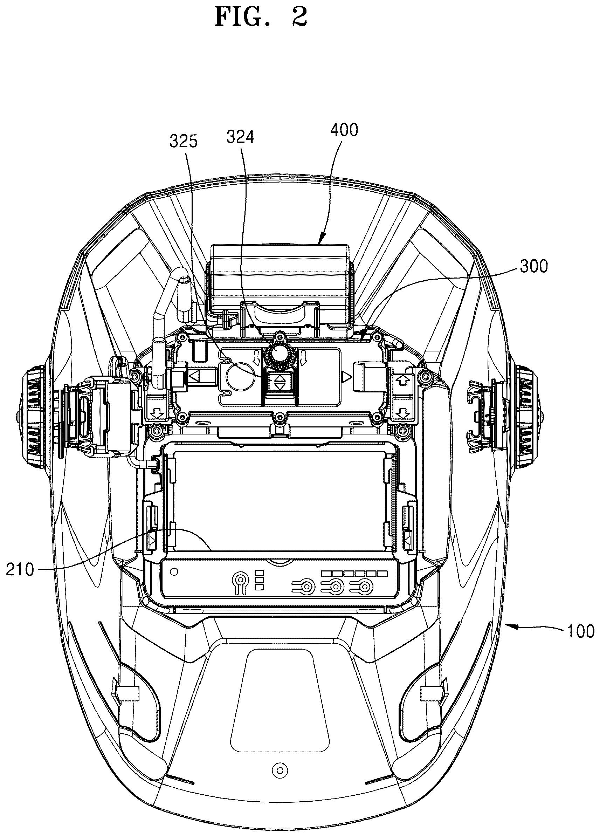

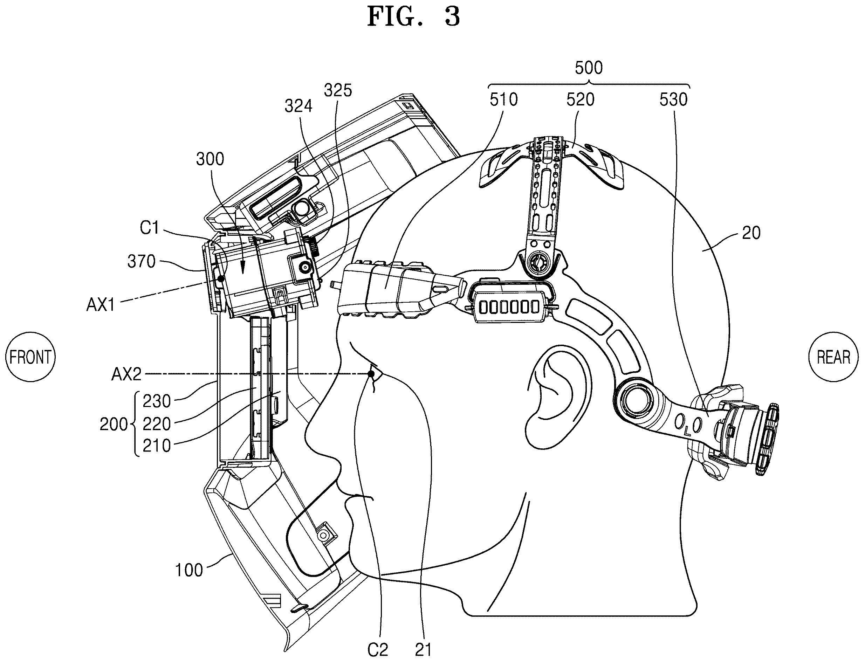

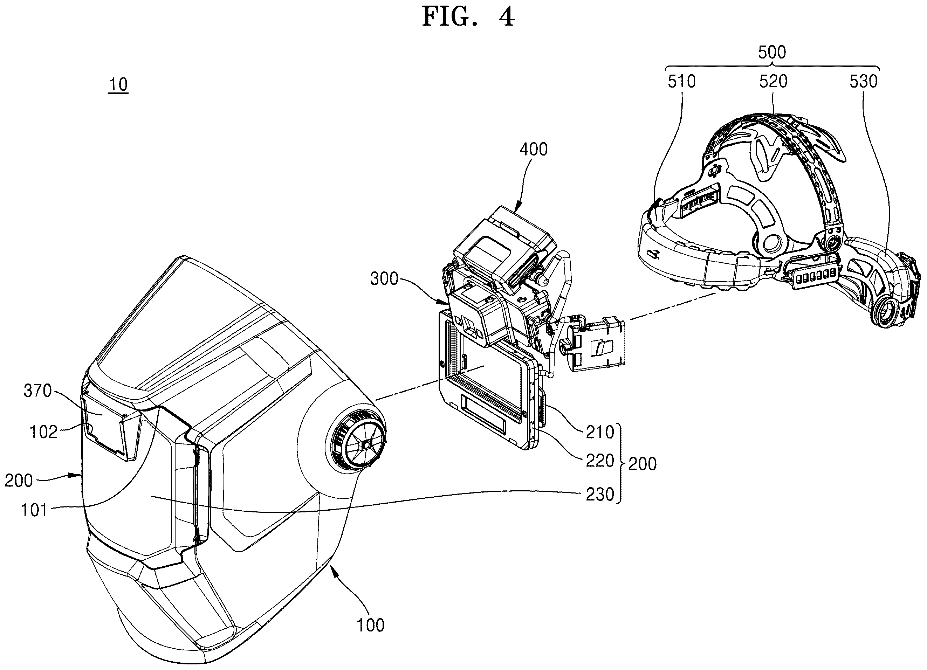

FIG. 1 is a perspective view illustrating a welding mask according to an embodiment of the present disclosure, FIG. 2 is a rear view of FIG. 1, FIG. 3 is a schematic side view illustrating a state in which a user wears the welding mask of FIG. 1, and FIG. 4 is a partial exploded perspective view of FIG. 1.

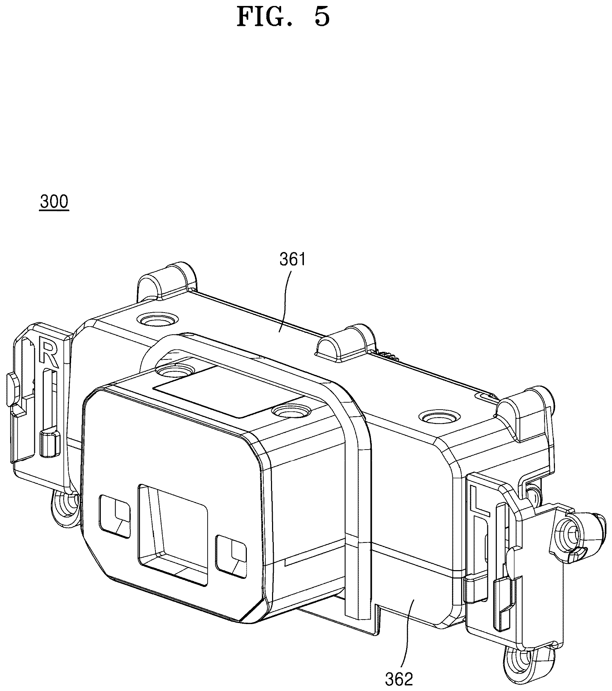

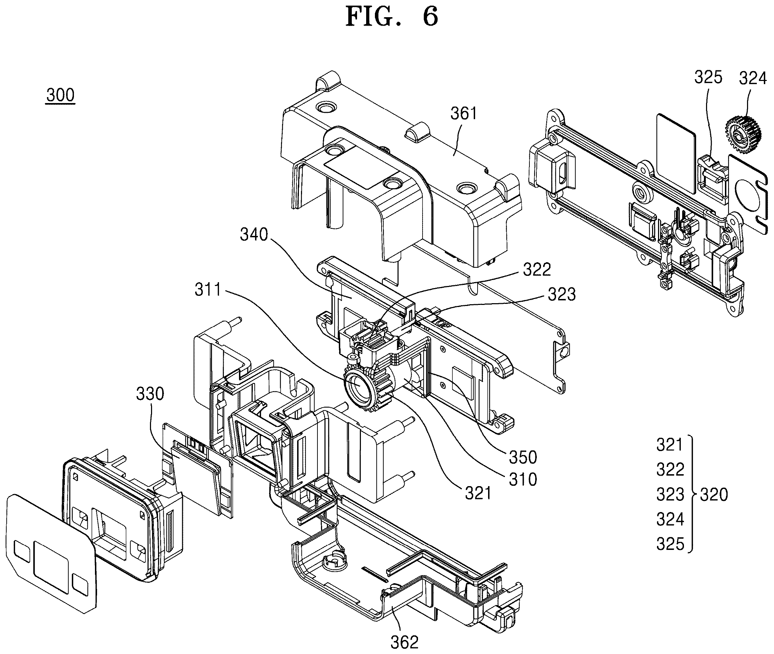

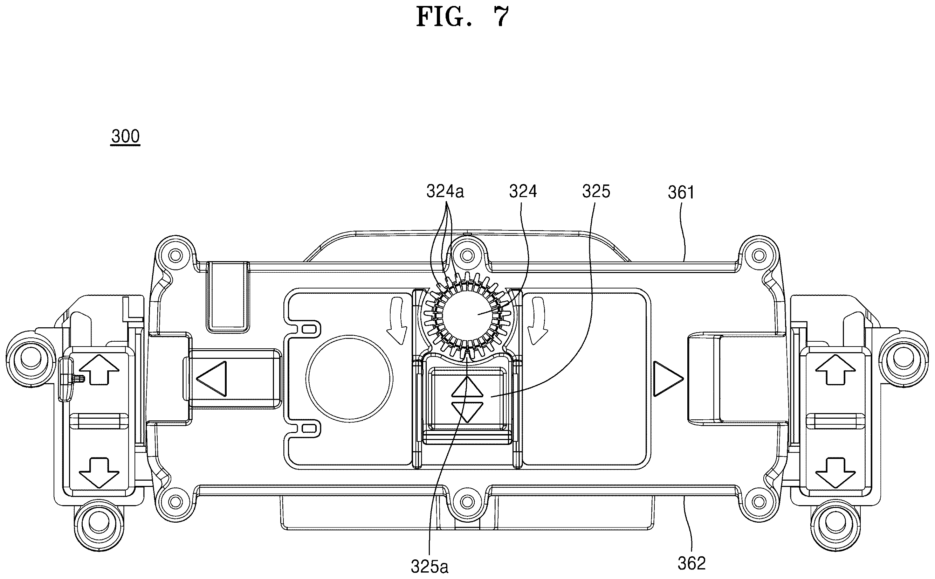

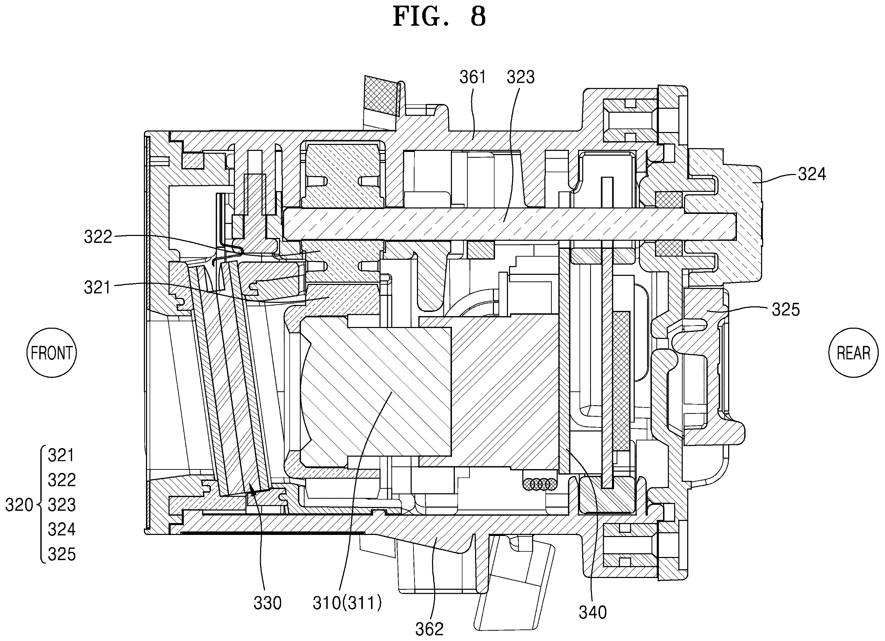

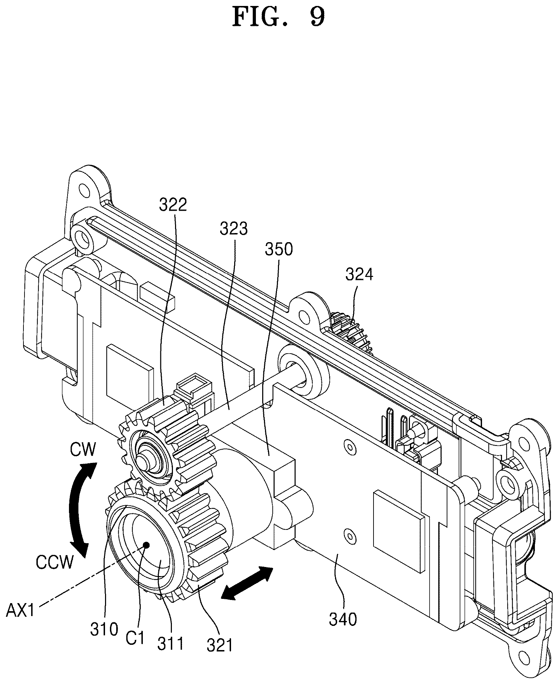

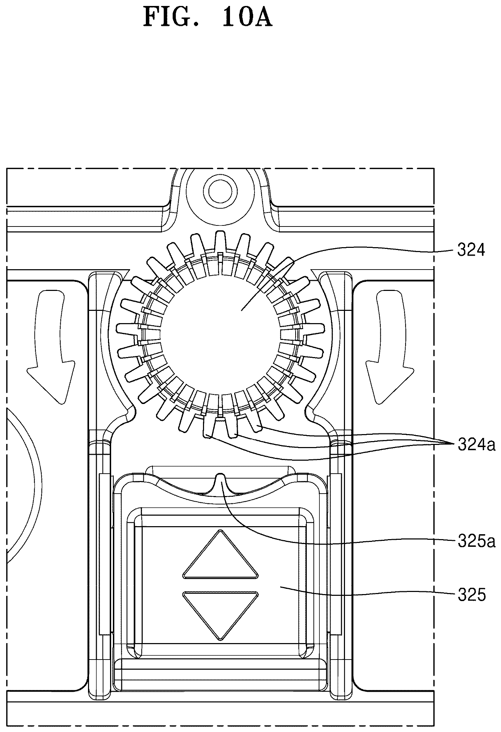

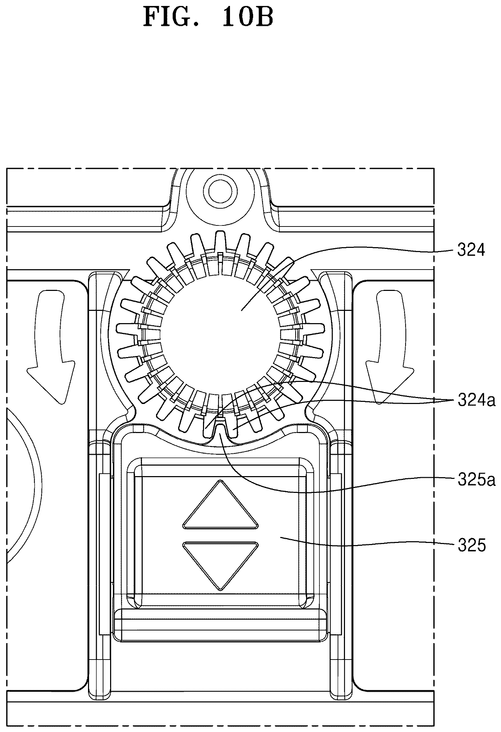

FIG. 5 is a perspective view illustrating a camera module of FIG. 4, FIG. 6 is an exploded perspective view of FIG. 5, FIG. 7 is a rear view of FIG. 5, FIG. 8 is a cross-sectional view taken along line I-I' of FIG. 5, FIG. 9 is a diagram partially illustrating a camera module of FIG. 5, FIG. 10A is a diagram illustrating region 'A' of FIG. 7, and FIG. 10B is a diagram illustrating a state in which rotational operation of an operating element is restricted by a locking unit of FIG. 10A.

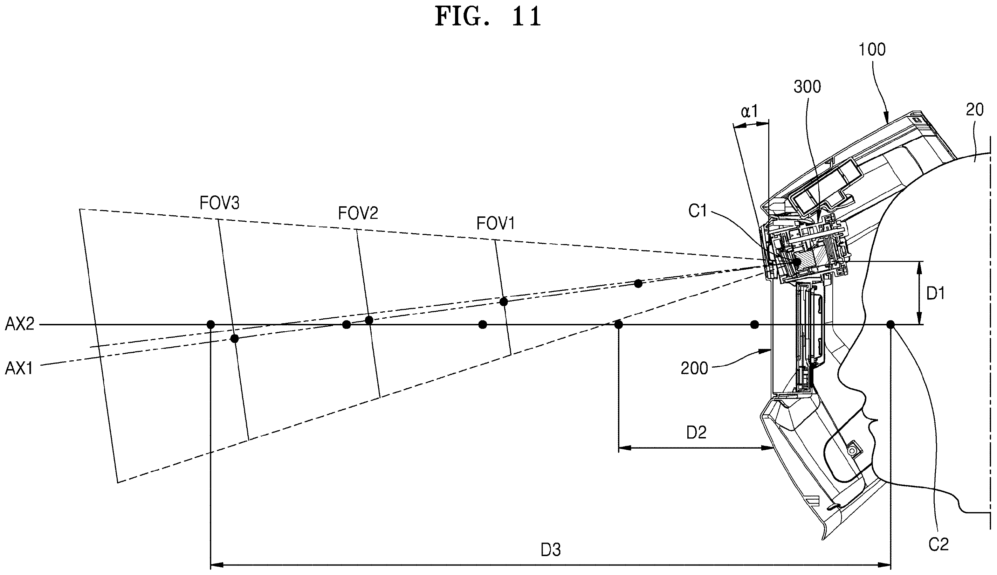

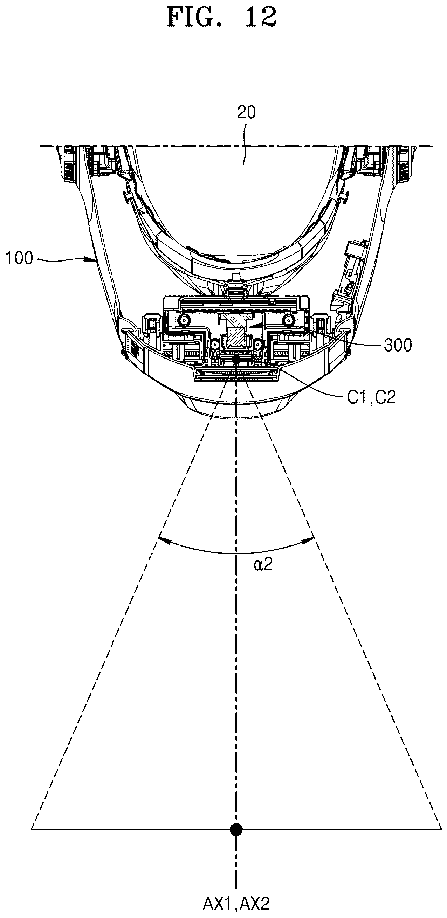

In addition, FIG. 11 is a diagram for describing the relationship between a vertical angle of view of a camera module and a view of a user, according to an embodiment of the present disclosure, and FIG. 12 is a diagram illustrating a horizontal angle of view of a camera module, according to an embodiment of the present disclosure.

Referring to FIGS. 1 to 4, a welding mask 10 according to an embodiment of the present disclosure may include a main body 100, a viewing window 200, a camera module 300, a battery 400, and a head gear 500.

The welding mask 10 may include the viewing window 200 in the main body 100, which enables high optical clarity and easily ensures a view, thereby providing a safe and stable view to the user 20 during welding work and improving work efficiency.

The welding mask 10 includes, on the main body 100, the camera module 300 having an optical center axis at a predetermined angle with respect to a viewing center axis of the user, and thus, optimized images in various welding environments may be obtained and various uses such as quality monitoring of welding work, provision of educational videos, and management of work status may be provided.

The focus of the camera module 300 may be manually adjusted from the inside of the main body 100 of the welding mask 10, and the welding mask 10 may be locked with the focus of the camera module 300 adjusted. Accordingly, images optimized for short and long distances according to an actual view of the user 20 and the style of the user 20 may be obtained, and stable images may be obtained by maintaining a fixed focus without focus shake even when subjected to vibration or external shock during welding work.

According to the welding mask 10, workability, such as convenience and continuity of use, may be increased by supplying power to components such as the camera module 300, through the battery 400, which is detachable, in the main body 100.

The welding mask 10 may be uniformly supported and fixed over the entire head of the user 20 through the head gear 500, thereby providing stability when worn with uniform load distribution, reducing user fatigue, and increasing work efficiency.

The main body 100 may be worn on the face of the user 20 to protect the entire face of the user 20 from light, high heat, spatter, or the like generated during welding work, and may have a hard shell shape in which the front and back thereof are formed into soft curves to fit the curvature of the human face.

The main body 100 may include high-impact nylon (polyamide (PA)) or polycarbonate (PC) with excellent heat resistance and impact resistance, but is not limited thereto, and may include various materials with heat resistance and impact resistance to protect the entire face of the user 20 from light, high heat, spatter, etc.

A viewing opening 101 in which the viewing window 200 is arranged to secure a view of the user 20 may be formed in the main body 100, and a camera opening 102 in which the camera module 300 is arranged may be formed above the viewing opening 101. The camera opening 102 may protrude forward more than the viewing opening 101, and a cover plate 370 to be described later may be provided to protect the camera module 300.

Referring to FIGS. 1 to 4, the viewing window 200 may be positioned in the viewing opening 101 of the main body 100 to secure a view corresponding to the left and right eyes 21 of the user 20, and may include a magnifying glass unit 210, a light irradiation unit 220, and a cover unit 230.

The magnifying glass unit 210 may include a magnifying lens provided in a square frame, and the user 20 may view a welding portion corresponding to a subject clearly and large.

The magnifying lens applied to the magnifying glass unit 210 may include a variety of magnifying lenses, such as a fixed-focus convex lens, a prism-type magnifying lens using a prism structure, and a thin and light multi-step structured Fresnel lens, but is not limited thereto.

Through the magnifying glass unit 210, the user 20 may precisely observe fine defects such as cracks, pores, and undercuts in welding beads of the welding portion during welding work, and may perform efficient work even in narrow spaces and narrow fields of view.

The light irradiation unit 220 may be arranged in front of the magnifying glass unit 210, and for example, an auto darkening filter (ADF) may be applied as a darkening filter. Although not shown in detail, the ADF may include an optical filter cell, an optical cover glass, and upper and lower polarizer housings.

The optical filter cell of the ADF may include a liquid crystal layer that controls light transmittance by changing the arrangement of liquid crystal molecules located between polarizers, according to an electric signal, polarizers that block light when the liquid crystals are not driven and transmit light when driven with a cross-polarization structure, and a thin film transistor (TFT) matrix that supplies electric signals to the liquid crystal display (LCD) cells.

The optical cover glass of the ADF may have impact resistance and transparency, and may include tempered glass or transparent polycarbonate to protect the liquid crystal layer.

The upper and lower polarizer housings of the ADF may include a metal or reinforced plastic frame that securely supports the entire ADF assembly.

The ADF may include a light sensor (not shown), which may detect the brightness of a welding arc to determine a driving timing of the light irradiation unit 220.

Due to this, in a standby state where the power is cut off or deactivated, the liquid crystal molecules are randomly arranged in the light irradiation unit 220 to block light transmission, thereby preventing excessive light from entering during preparation for welding work.

In addition, when the light sensor detects light of a welding arc in a light-blocking state during welding work, the light irradiation unit 220 may adjust voltage applied to the polarizers to align the liquid crystal molecules along the axis of the polarizers, thereby rapidly reducing the light transmittance and blocking arc light.

In addition, as the arc is extinguished and the liquid crystal molecules return to their original arrangement to increase the light transmittance in a transmitted state when the work is completed, the light irradiation unit 220 may provide a clear view to the user without color distortion.

The cover unit 230 may protect the light irradiation unit 220 and the magnifying glass unit 210 from external impact, high heat, spatter, etc., and may provide a high-resolution, safe, and stable view to the user 20 by further including various functions.

For example, the cover unit 230 may have a function of high definition view (HDV), and for this purpose, the cover unit 230 may have a multilayer laminate structure in which a plurality of layers such as an outermost cover layer, an intermediate HDV core layer, and a packing layer are stacked.

The outermost cover layer may include optical polycarbonate or tempered optical glass, and may be hard coated to prevent scratches, etc.

Additionally, the intermediate HDV core layer may include an ultra-thin transparent optical film having a preset refractive index, specifically, a polymethyl methacrylate (PMMA)-based high-refractive-index film.

In addition, the packing layer may include silicone or ethylene propylene diene terpolymer (EPDM) rubber seal for dustproofing and waterproofing, and may seal the front edge of the cover unit 230, i.e., the viewing opening 101, thereby blocking dust and moisture from entering the inside of the light irradiation unit 220 and the magnifying glass unit 210.

A surface of the cover unit 230 may be coated. For example, the surface of the cover unit 230 may be treated with an anti-reflective (AR) coating to reduce external reflectivity and minimize glare in the view of the user 20.

In addition, the surface of the cover unit 230 may be treated with an anti-scratch coating to have a hardness of 7H or higher, thereby preventing physical damage such as surface scratches.

In addition, the surface of the cover unit 230 may be treated with an anti-fog coating to prevent fogging, and treated with an anti-static coating to prevent static electricity generation, thereby preventing foreign substances such as dust from attaching and becoming contaminated.

Referring to FIGS. 5 to 9, the camera module 300 may be mounted on the main body 100 to capture an image of a welding operation located in the view of the user 20, and may include a lens barrel 310, a focus adjustment unit 320, an ADF 330, a substrate 340, an image sensor 350, upper and lower housings 361 and 362, and a cover plate 370.

The lens barrel 310 may have a lens unit 311 which includes a plurality of lenses, such as a block lens, a concave lens, and an aspherical lens, and is fixedly supported therein, and may be formed in a cylindrical shape such that spacing between the plurality of lenses and an optical center axis AX1 are accurately maintained.

The lens barrel 310 may be rotatably arranged in the upper and lower housings 361 and 362 described later, and may be rotated clockwise (CW) or counterclockwise (CCW) by operating the focus adjustment unit 320.

By rotating the lens barrel 310 clockwise (CW) or counterclockwise (CCW), the lens unit 311 may be moved back and forth in a straight line along the optical center axis AX1, and the focus of the camera module 300 may be adjusted by changing a distance between the lens unit 311 and the image sensor 350 described later.

An inner surface of the lens barrel 310 may be black-coated to absorb unnecessarily scattered light, and may include a highly rigid material such as aluminum alloy, stainless steel, or polyoxymethylene (POM) to protect the lens unit 311 from shock and vibration.

The focus adjustment unit 320 may be used to manipulate the clockwise (CW) or counterclockwise (CCW) rotation of the lens barrel 310, and may have one side connected to the lens barrel 310 and the other side that is connected to the one side and exposed to the inside of the main body 100, and the lens barrel 310 may be rotated by manipulating the exposed other side.

The focus adjustment unit 320 may include a first gear 321, a second gear 322, a shaft 323, an operating element 324, and a locking unit 325.

The first gear 321 may be coupled to the front outer surface of the lens barrel 310 and rotate together with the lens barrel 310. The first gear 321 may include a spur gear and rotate clockwise (CW) or counterclockwise (CCW) in the same manner as the lens barrel 310.

The second gear 322 may be engaged with the first gear 321 and rotate together with the first gear 321. The second gear 322 may include a spur gear and rotate in the opposite direction to the first gear 321.

That is, when the second gear 322 rotates clockwise (CW), the first gear 321 may rotate counterclockwise (CCW), and when the second gear 322 rotates counterclockwise (CCW), the first gear 321 may rotate clockwise (CW).

The front end of the shaft 323 may be coupled to the second gear 322, and the rear end of the shaft 323 may be coupled to the operating element 324 described later. Accordingly, when the operating element 324 is rotated, the second gear 322 connected to the shaft 323 may be rotated, and when the second gear 322 is rotated, the first gear 321 and the lens barrel 310 may be rotated.

The operating element 324 may be connected to the rear end of the shaft 323 and rotate together with the second gear 322. The operating element 324 may be exposed to the inside of the main body 100, that is, toward the face of the user 20, and may be rotated by the user 20.

A plurality of gear teeth 324a may be formed continuously on an outer circumferential surface of the operating element 324, and the user 20 may easily grip and rotate the operating element 324 by the plurality of gear teeth 324a.

The locking unit 325 may be arranged on one side of the operating element 324, specifically below the operating element 324, to restrict rotation of the operating element 324. For example, a locking projection 325a that may be inserted between the plurality of gear teeth 324a of the operating element 324 may be formed in an upper portion of the locking unit 325, and the locking unit 325 may be linearly moved back and forth in the radial direction of the operating element 324 (vertical direction based on FIG. 7).

Referring to FIG. 10A, when the locking unit 325 moves linearly and downward in the radial direction of the operating element 324, the locking projection 325a may come out from between the plurality of gear teeth 324a, and the operating element 324 may be in a rotatable state.

Referring to FIG. 10B, when the locking unit 325 moves linearly and upward in the radial direction of the operating element 324, the locking projection 325a may be inserted between the plurality of gear teeth 324a, and accordingly, the operating element 324 may be in a state in which the rotational operation thereof is locked.

In the welding mask 10 of the present embodiment, the focus of the camera module 300 may be manually adjusted from the inside of the main body 100 through the focus adjustment unit 320. Accordingly, an image optimized for short and long distances according to the actual view of the user 20 and the style of the user 20 may be obtained, and the focus state may be quickly changed according to the welding posture or work target.

In addition, the welding mask 10 of the present embodiment may be locked in a focus-adjusted state after adjusting the focus of the camera module 300 through the focus adjustment unit 320. Thus, stable images may be obtained by maintaining a fixed focus without focus shake even when subjected to vibrations or external shocks during welding work, and images may be captured quickly without the need for focus adjustment each time work is performed at a similar distance.

Referring to FIGS. 5 to 9, the ADF 330 of the camera module 300 may protect the image sensor 350 from a welding arc and adjust the exposure settings of the camera module 300 to stably secure high-quality images.

The ADF 330 of the camera module 300 may include LCD cells, a light sensor, and a driving circuit, and may lower the light transmittance to protect the image sensor 350 when light exceeding a threshold is detected, may lower the exposure in a dark state to prevent excessive exposure of the image sensor 350, and may return to normal transmittance when an arc is not detected.

A similar configuration and method to those of the ADF applied to the light irradiation unit 220 of the viewing window 200 described above may be applied to the ADF 330 of the camera module 300, and thus a detailed description thereof will be omitted for convenience.

The substrate 340 may be fixedly arranged within the upper and lower housings 361 and 362 and act as an electrical interconnector that distributes electrical signals and power between components constituting the camera module 300.

For example, the substrate 340 may include a printed circuit board (PCB) in which the image sensor 350, an ADF control unit, and a control/processing circuit are integrated and mounted on a single board.

The substrate 340 may provide a location at which the image sensor 350 is fixed on the camera module 300, and the lens barrel 310 and the lens unit 311 may be arranged on a front portion of the substrate 340, and the operating element 324 and the locking unit 325 of the focus adjustment unit 320 may be arranged on a rear portion of the substrate 340.

The image sensor 350 may be a complementary metal oxide semiconductor (CMOS) sensor or a charge coupled device (CCD) sensor, and in the present embodiment, a global shutter CMOS sensor having high resolution, high sensitivity, high frame rate, etc. may be applied, but is not limited thereto.

The upper and lower housings 361 and 362 may be coupled to each other to form the exterior of the camera module 300, provide a space for installing various optical and electrical components that constitute the camera module 300, and may protect the various installed components.

The cover plate 370 may be provided in the camera opening 102 of the main body 100 to protect the camera module 300 from external impact, high heat, spatter, etc., and have various functions added to enable the camera module 300 to obtain an optimized image.

A similar configuration and method to those applied to the cover unit 230 of the viewing window 200 may be applied to the cover plate 370, and thus a detailed description thereof will be omitted for convenience.

Referring to FIG. 11, the camera module 300 of the present embodiment may be arranged above the viewing window 200, and may be arranged such that the optical center axis AX1 has a certain angle with a viewing center axis AX2.

A preset angle between the optical center axis AX1 and the viewing center axis AX2 may be an angle α1 inclined downward and forward while the front surface of the camera module 300 is vertical. The angle α1 may have a range of about 10 degrees to about 16 degrees, and specifically, may have an angle of 13 degrees.

In addition, in the present embodiment, an optical center C1 of the camera module 300 and a viewing center C2 of the left and right eyes 21 of the user 20 may be arranged to have a predetermined distance D1 therebetween. The distance D1 between the optical center C1 and the viewing center C2 may have a range of about 40 mm to about 50 mm, and specifically, a distance of 45 mm.

Accordingly, regarding the welding mask 10 of the present embodiment, a minimum distance which is included in the view of the user 20 and at which an image may be captured by the camera module 300, specifically, a distance D2 from the viewing window 200 to a minimum point at which an image may be captured, may be approximately 100 mm. That is, a distance from the viewing center C2 to the minimum point at which image capturing is easily performed may be about 200 mm.

In addition, a maximum distance which is included in the view of the user 20 and at which an image may be captured by the camera module 300, specifically a horizontal distance D3 to a point at which the optical center axis AX1 and the viewing center axis AX2 intersect each other, may be about 500 mm. That is, the horizontal distance from the viewing center C2 to a point where the center of the optical center C1 of the camera lens and the viewing center C2 theoretically correspond to each other may be 500 mm. This is because, considering the typical arm length of the user 20, welding work is possible up to a distance of 500 mm from the viewing center C2 and an optimized image may be obtained up to this distance.

Referring again to FIG. 11, the camera module 300 of the present embodiment may change the size of a field of view FOV for obtaining a clear image by adjusting the focus on a welding portion.

By manipulating the focus adjustment unit 320 to move the lens barrel 310 as far back as possible, the distance between the lens unit 311 and the image sensor 350 may be brought as close as possible. A maximum field of view FOV3 of an image that may be obtained by the camera module 300 may have a size of, for example, 315 mm × 164 mm (width × height). That is, the camera module 300 may capture an image of a wide area beyond the welding portion by enlarging the surrounding region.

Additionally, by operating the focus adjustment unit 320 to move the lens barrel 310 forward as much as possible, a gap between the lens unit 311 and the image sensor 350 may be maximized. A minimum field of view FOV1 of an image that may be obtained by the camera module 300 may have a size of, for example, 163 mm × 84 mm (width × height). That is, the camera module 300 may capture an image by enlarging a center of the welding portion with a relatively narrow width.

Additionally, by manipulating the focus adjustment unit 320 to position the lens barrel 310 at a middle position (neutral) between maximum forward movement and maximum backward movement thereof, the distance between the lens unit 311 and the image sensor 350 may be positioned at the middle position. A size of a medium field of view FOV2 of an image that may be obtained by the camera module 300 may have a size of, for example, 293 mm x 124 mm (width x height). That is, the camera module 300 may capture, with a standard width, balanced images of a welding portion.

In other words, according to the welding mask 10 of the present embodiment, an image having various screen sizes may be obtained using the camera module 300 by adjusting the focus adjustment unit 320. For example, in the maximum field of view FOV3, images of a wide range of backgrounds and surroundings along with the welding portion may be obtained, in the medium field of view FOV2, images of the entire welding portion and some surroundings may be obtained, and in the minimum field of view FOV1, images of detailed areas such as the bead shape, surface defects, and spatter of the welding portion may be obtained.

Referring to FIG. 12, a range of an angle α2 of the horizontal direction (left-right direction based on FIG. 12) of the camera module 300 of the present embodiment may be similar to the range of the horizontal direction (left-right direction based on FIG. 12) of the view of the user 20.

The optical center axis AX1 of the camera module 300 and the viewing center axis AX2 of the user 20 may correspond to each other.

An image of welding work, obtained by the camera module 300 of the present embodiment, may be stored in a separate storage and transmitted externally in real time, thereby enabling various uses such as quality monitoring of the welding work, provision of educational videos, and management of the work status.

Referring to FIGS. 2 to 4, the battery 400 may be detachably coupled to one side, specifically the upper side, of the camera module 300, to supply power to the camera module 300 and, in addition, to supply power to various electrical components such as the light irradiation unit 220 of the viewing window 200.

In the present embodiment, the battery 400 may be detachably mounted to the main body 100, thereby improving continuity of operation, convenience of use, and maintainability.

For example, during welding work, only the battery 400 may be quickly replaced, allowing work to be quickly resumed without interruption, and by carrying a charged spare battery 400, work downtime due to lack of power may be minimized even during shift work or long-term work.

In addition, by applying the detachable structure of the battery 400 to various locations such as a side of the main body 100, the insertion or detachment of the battery 400 may be performed quickly and easily without obstructing the posture or view of the user 20.

In addition, the detachable structure of the battery 400 may facilitate maintenance and reduce repair costs, and the reliability of the welding mask 10 may be improved by allowing for easy replacement when performance of the battery 400 deteriorates.

Referring to FIGS. 1, 3, and 4, the head gear 500 may be removably attached to the head of the user 20, and the user 20 may wear or remove the welding mask 10 via the head gear 500.

The head gear 500 may include a frontal strap 510, a parietal strap 520, and an occipital strap 530, and may be coupled to both upper sides of the main body 100. The head gear 500 may be, while being connected to the main body 100, rotated forward or backward, and the user 20 may rotate the head gear 500 to adjust the viewing window 200 to face exactly the front of the left and right eyes 21.

The frontal strap 510 may be supported and fixed to the forehead of the user 20, the parietal strap 520 may be supported and fixed to the crown of the head of the user 20, and the occipital strap 530 may be supported and fixed to the back of the head of the user 20.

When worn on the head of the user 20, the head gear 500 of the present embodiment may be uniformly supported and fixed over the entire head including the front, upper, and back surfaces, and thus, the user 20 may wear the same with considerable stability during welding work, and fatigue of the user 20 may be reduced through uniform load distribution and work efficiency may be increased.

While the present disclosure has been particularly shown and described with reference to embodiments thereof, it will be understood by those of ordinary skill in the art that various changes in form and details may be made therein without departing from the spirit and scope as defined by the following claims. Therefore, the scope sought to be protected of the present disclosure shall be defined by the appended claims.

A welding mask according to an embodiment of the present disclosure may provide a stable and accurate view to a user during welding work to improve welding accuracy, and may enhance usability by capturing, recording, and transmitting images of the welding work in real time.

In addition, the user may adjust the camera focus to the actual view to obtain an optimized image, and the adjusted focus may be locked and maintained to prevent focus shake, and may thus have improved workability in similar work.

The scope of the present disclosure is not limited by these effects.

## Claims

1. A welding mask comprising:
a main body that is wearable on the face of a user;
a viewing window disposed on the main body and corresponding to the left and right eyes of the user; and
a camera module mounted on the main body and configured to capture an external image located in a view of the user,
wherein the camera module comprises:
a lens barrel in which a lens unit is built; and
a focus adjustment unit having one side connected to the lens barrel and the other side connected to the one side and exposed to an inside of the main body, the focus adjustment unit being configured to move the lens unit along an optical center axis as the lens barrel is rotated by manipulating the other side.

2. The welding mask of claim 1, wherein the focus adjustment unit comprises:
a first gear coupled to an outer circumferential surface of the lens barrel;
a second gear engaged with the first gear; and
an operating element connected to the second gear via a shaft, exposed to the inside of the main body, and being rotatable by the user.

3. The welding mask of claim 2, wherein the first gear and the second gear each comprise a spur gear, and the first gear and the second gear rotate in opposite directions to each other.

4. The welding mask of claim 2, wherein the camera module further comprises a locking unit arranged on one side of the operating element to restrict rotation of the operating element.

5. The welding mask of claim 4, wherein a plurality of gear teeth are continuously formed on an outer circumferential surface of the operating element, and
the locking unit has a locking projection that is insertable between a plurality of gear teeth, and the locking unit is movable linearly back and forth in a radial direction of the operating element.

6. The welding mask of claim 1, wherein the camera module is positioned above the viewing window, and
an optical center axis of the camera module and a viewing center axis of the viewing window have a preset angle therebetween.

7. The welding mask of claim 1, wherein an optical center of the camera module and a viewing center of the left and right eyes of the user have a preset distance therebetween.

8. The welding mask of claim 1, further comprising a battery detachably coupled to one side of the camera module and configured to supply power to the camera module.

9. The welding mask of claim 1, wherein the viewing window comprises:
a magnifying glass unit corresponding to the left and right eyes of the user;
a light irradiation unit positioned in front of the magnifying glass unit; and
a cover unit arranged in front of the light irradiation unit.
